(19) Europäisches Patentamt
European Patent Office
Office européen des brevets

(11) Veröffentlichungsnummer: **0 228 701 B1**

(12) **EUROPÄISCHE PATENTSCHRIFT**

(45) Veröffentlichungstag der Patentschrift: **27.02.91**

(51) Int. Cl.$^5$: **C07C 19/08**, C07C 17/04, H01S 3/20

(21) Anmeldenummer: **86118011.5**

(22) Anmeldetag: **23.12.86**

Die Akte enthält technische Angaben, die nach dem Eingang der Anmeldung eingereicht wurden und die nicht in dieser Patentschrift enthalten sind.

(54) **2-Iod-perfluor-2-methylalkane, Verfahren zu deren Herstellung und deren Verwendung.**

(30) Priorität: **04.01.86 DE 3600108**

(43) Veröffentlichungstag der Anmeldung: **15.07.87 Patentblatt 87/29**

(45) Bekanntmachung des Hinweises auf die Patenterteilung: **27.02.91 Patentblatt 91/09**

(84) Benannte Vertragsstaaten: **DE FR GB IT**

(56) Entgegenhaltungen:
**DE-C- 1 121 598**

**JOURNAL OF THE AMERICAN CHEMICAL SOCIETY, Band 83, Nr. 11, July 1961, Seiten 2409-2500; I.J. GARDNER et al.: "Effects of substituents on the radical exchange reaction between benzyl iodide and iodine"**

**JOURNAL OF ORGANIC CHEMISTRY, Band 32, Nr. 11, 1967, Seiten 1682-1683; J.A. YOUNG et al.: "The synthesis of perfluoroisobutylene"**

**W. R. Weaver und J. H. Lee, "Journal of Energy", 1983, Vol. 7, Nr. 6, S. 498 ff**

(73) Patentinhaber: **HOECHST AKTIENGESELLSCHAFT**
**Postfach 80 03 20**
**D-6230 Frankfurt am Main 80(DE)**

(72) Erfinder: **Probst, Anton**
**Gerhart-Hauptmann-Strasse 5**
**D-8269 Burgkirchen(DE)**
Erfinder: **Raab, Klaus, Dr.**
**Schneibsteinstrasse 6**
**D-8269 Burgkirchen(DE)**
Erfinder: **Werner von, Konrad, Dr.**
**Buch 1 1/2**
**D-8261 Halsbach(DE)**

EP 0 228 701 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 2-Iod-perfluor-2-methylalkane, deren Herstellung und Verwendung gemäß nachfolgenden Patentansprüchen.

Bisher ist nur 2-Iod-perfluor-2-methylpropan, das entsprechende Alkan mit dem niedrigsten Molekulargewicht, bekannt. Es ist eine feste, sehr flüchtige Substanz vom Schmelzpunkt 81 °C und kann beispielsweise nach J.A. Young und Th. M. Reed, J. Org. Chem. 32 (1967), Seite 1682, rechte Spalte, 2. Absatz, durch katalysierte Anlagerung der Elemente des Iodmonofluorids an das hochgiftige Perfluorisobutylen während 60 Stunden bei 130 °C hergestellt werden. Als Quelle für Iodmonofluorid wird von M. Hauptschein; A. H. Fainberg und M. Braid, J. Am. Chem. Soc. 80 (1958), Seite 842 ff. ein Gemisch von 2 mol Iod und 1 mol Iod Pentafluorid verwendet. Wie nachfolgende Vergleichsversuche A bis D zeigen, kann nach dieser Methode an Perfluor-2-methyl-2-buten oder Perfluor-2-methyl-2-penten kein Iodfluorid angelagert werden, auch dann nicht, wenn als Katalysator Metallfluoride wie Antimon(V)-fluorid oder Kobalt(III)-fluorid in beträchtlichen Mengen eingesetzt werden. Selbst wenn man je mol verwendetes Iodpentafluorid nur 1 mol Kaliumfluorid einsetzt, werden lediglich Spuren der Anlagerungsverbindung von Iodfluorid erhalten.

2-Iod-perfluor-2-methylpropan kann ferner nach E. P. Mochalina; B.L. Dyatkin; I. V. Galakhor und I.L. Knunyants; Dokl. Akad. Nauk.SSSR 169 (6) S 1346 - 1349 (1966) referiert in C.A. 66 (1967) Nr. 45901 Y durch Erhitzen von Perfluorisobutylen mit Iod,Kaliumfluorid und Nitrobenzol während 10 Stunden bei 170 bis 180 °C in einem Stahlautoklaven mit guter Ausbeute gewonnen werden. Wie nachfolgender Vergleichsversuch E zeigt, ist dieses Verfahren ebenfalls nicht geeignet, um 2-Iod-perfluor-2-methyl-butan oder das entsprechende Pentan durch Anlagerung von Iodfluorid an die entsprechenden perfluorierten Olefine darzustellen. Dies gelingt nach Vergleichsversuch F auch nicht, wenn das als Lösungsmittel verwendete Nitrobenzol weggelassen wird.

Es wurden nun Verfahren gefunden, um 2-Iod-perfluor-2-methyl-butan oder das entsprechende Pentan herzustellen. Gegenstand der Erfindung sind demnach Verbindungen der Formel

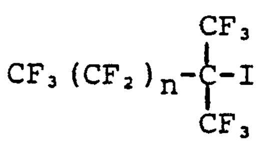

(I),

worin bedeutet n = 1 oder 2, sowie ein Verfahren zur Herstellung von Verbindungen der Formel (I) durch Umsetzung von perfluorierten, verzweigtkettigen Olefinen mit einer Mischung aus Iod und Iodpentafluorid im Molverhältnis von 2 : 1 bei erhöhter Temperatur unter dem autogenen Druck des Reaktionsgemisches oder einem darüberliegenden Druck, das dadurch gekennzeichnet ist, daß als perfluoriertes, verzweigtkettiges Olefin Perfluor-2-methyl-2-buten oder Perfluor-2-methyl-2-penten in einer Menge von mindestens 5 mol je mol verwendetes Iodpentafluorid eingesetzt wird und die Umsetzung in Gegenwart von mehr als 1 mol je mol verwendetes Iodpentafluorid von mindestens einem Fluorid eines Metalls der I. Gruppe des Periodensystems der Elemente durchgeführt wird.

Perfluor-2-methyl-2-buten kann beispielsweise erhalten werden durch Isomerisierung von Perfluor-2-methyl-3-buten, das nach R. N. Haszeldine et al., J. Chem.Soc. Perkin I/1973, Seite 574, hergestellt wird. Die Isomerisierung ist beschrieben bei R. N. Haszeldine et al., Chem. Commun. 1970, S 1444 ff. Dort ist auch die Herstellung von Perfluor-2-methyl-2-penten beschrieben. Jede der beiden Verbindungen wird in einer Menge von mindestens 5 mol, je mol verwendetes Iodpentafluorid ($IF_5$),eingesetzt. Werden je mol $IF_5$ weniger als 5 mol der genannten Verbindungen verwendet, so werden die 2-Iod-perfluor-2-methyl-alkane der Formel (I) nur in geringerer Ausbeute erhalten. Es können ohne Nachteil mehr als 5 mol Perfluor-2-methyl-2-buten oder des entsprechenden -penten je mol $IF_5$ eingesetzt werden, da diese Ausgangsprodukte bei der im Anschluß an die Reaktion erfolgenden Aufarbeitung durch Destillation leicht von den Endprodukten der Formel (I) abgetrennt werden können, weil die Siedepunkte der Perfluorolefine deutlich niedriger liegen als die der Iodide. Lediglich aus wirtschaftlichen Gründen, um nicht eine unnötig hohe Menge der nicht-umgesetzten verwendeten Ausgangsprodukte wiedergewinnen zu müssen, wird man 10 mol Perfluoroolefine je mol $IF_5$ nicht überschreiten. Die Reaktion wird ferner in Gegenwart von mehr als 1 mol je mol verwendetes $IF_5$ von mindestens einem Fluorid eines Metalles der I. Gruppe des Periodensystems der Elemente durchgeführt. Geeignet sind beispielsweise Natriumfluorid, Lithiumfluorid, Rubidiumfluorid, Kupfer-(I)fluorid und Silberfluorid. Geeignet sind auch Systeme, die die genannten Fluoride bilden, beispielsweise eine Mischung von feinverteiltem metallischem Kupfer und Kupfer(II)fluorid. Besonders gute Ergebnisse werden mit Kaliumfluorid und Cäsiumfluorid erhalten.

Die genannten Fluoride können nach bekannten Verfahren aktiviert werden, beispielsweise mit Schwefeldioxid, wie beschrieben von F. Seel und H. D. Görlitz; Z. f. Anorg. Allg. Chem. 327 (1964) Seite 32, oder durch Gefriertrocknung, siehe N. Tshikawa; T. Kitazume und M. Nakabayashi, Chem. Lett. 1980 , Seite 1089, ferner auch durch Vakuumtrocknung bzw. mit Hilfe von Perfluoraceton, wie beschrieben bei G. A. Kolta; G. Webb und J. M. Winfield, J. Fluorine Chem. 14 (1979), Seite 331. Im allgemeinen ist jedoch eine besondere Aktivierung nicht erforderlich.

Es können auch Mischungen von verschiedenen Metallfluoriden der I. Gruppe des Periodensystems der Elemente verwendet werden.

Von den im vorangegangenen Absatz beschriebenen Fluoriden werden mehr als 1 mol je mol $IF_5$ eingesetzt. Wird das Metallfluorid nicht im molaren Überschuß verwendet, werden nur geringe Mengen der Verbindungen der Formel (I) erhalten, wie der nachfolgende Vergleichsversuch B zeigt. Nach oben ist die Menge des verwendeten Metallfluorids im wesentlichen durch wirtschaftliche Erwägungen begrenzt, über 5 mol Metallfluorid je mol $IF_5$ wird kein zusätzlicher Effekt mehr festgestellt, vorzugsweise wird die Umsetzung des Perfluorolefins mit der Mischung aus lod und $IF_5$ in Gegenwart von 1,1 bis 4 mol Metallfluorid je mol verwendetes $IF_5$ durchgeführt.

Bei dem weiter oben näher beschriebenen erfindungsgemäßen Verfahren soll die Reaktionstemperatur vorzugsweise zwischen 170 und 220 °C liegen. Unter 170 °C vermindert sich die Reaktionsgeschwindigkeit deutlich, so daß unnötig lange Reaktionszeiten erforderlich werden, über 220 °C wird in zunehmenden Maße die Bildung unerwünschter Nebenprodukte festgestellt. Besonders gute Ergebnisse werden erhalten, wenn die Reaktionstemperatur zwischen 180 und 210 °C gehalten wird.

Die Dauer der Reaktion hängt von der gewählten Reaktionstemperatur ab, sie liegt vorteilhaft bei Temperaturen über 200 °C zwischen 2 und 12 h, bei Temperaturen im Bereich von 170 °C kann sie bis zu 30 h betragen, gute Ergebnisse werden bei einer Dauer der Reaktion von 7 bis 15 h erhalten.

Die Umsetzung wird unter dem autogenen Druck des Reaktionsgemisches im allgemeinen je nach Temperatur zwischen 2,2 und 2,7 MPa vorgenommen, höhere Drucke, wie sie beispielsweise durch Anwesenheit oder Zugabe von Inertgasen, beispielsweise Argon oder Stickstoff, auftreten können, beeinflussen das erfindungsgemäße Verfahren nicht negativ. Während der Reaktion wird zweckmäßig der Inhalt des Reaktionsgefäßes ständig in Bewegung gehalten, beispielsweise durch Rühren oder Schütteln. Alle angewendeten Reagenzien müssen vollständig wasserfrei sein, auch während der Beschickung des Reaktionsgefäßes ist die Einwirkung von Wasserdampf durch Anwendung einer trockenen Inertgas-Schutzatmosphäre auszuschließen. Als Reaktionsgefäß eignen sich Autoklaven aus chemikalienbeständigem Chrom-Nickelstahl.

Nach Ablauf der gewählten Reaktionsdauer wird das Reaktionsgefäß auf Raumtemperatur abgekühlt, langsam entspannt und die verbliebenen flüssigen und festen Anteile in eine zweckmäßig auf -3 bis +6 °C gekühlte 1 bis 20 gew.-%ige wäßrige Lösung eines Alkalihydroxids, beispielsweise Natriumhydroxid, eingerührt. Es bilden sich hierbei 2 Phasen, die organische Phase wird abgetrennt, mit möglichst kaltem Wasser gewaschen, anschließend mit einem geeigneten festen Trockenmittel, beispielsweise Magnesiumsulfat, getrocknet und bei Normaldruck fraktioniert destilliert. Sofern Perfluor-2-methyl-2-buten als Ausgangsprodukt eingesetzt wurde, gehen nicht-umgesetzte Anteile dieser Verbindung bei etwa 27 °C über und werden in einer mit Kühlsole gekühlten Vorlage aufgefangen. Die Hauptfraktion besteht aus 2-lod-perfluor-2-methyl-butan von hoher Reinheit und wird bei 89 bis 90 °C als violette Flüssigkeit abdestilliert. Sofern Perfluor-2-methyl-2-penten als Ausgangsprodukt verwendet wurde, geht das nicht-umgesetzte Olefin bei etwa 51 °C über. Das 2-lod-perfluor-2-methyl-pentan wird ebenfalls in hoher Reinheit als violette Flüssigkeit bei 115 °C abdestilliert.

Achtung! Wie das bekannte 2-lod-perfluor-2-methyl-propan sind auch die beim erfindungsgemäßen Verfahren gewonnenen Verbindungen der Formel (I) stark giftige Substanzen, die eine beträchtliche Flüchtigkeit aufweisen, auch die eingesetzten perfluorierten Olefine sind giftig, weshalb die gesamten Arbeiten zur erfindungsgemäßen Darstellung der Verbindungen der Formel (I) unter entsprechenden Schutzmaßnahmen, insbesondere Absaugung und Vernichtung entweichender Gase, durchzuführen sind.

Gegenstand der Erfindung ist ferner ein Verfahren zur Herstellung von Verbindungen der Formel (I) durch Umsetzung von perfluorierten, verzweigten Olefinen mit lod und einem Alkalimetallfluorid in Gegenwart eines aprotischen Lösungsmittels, gegebenenfalls bei erhöhter Temperatur, das dadurch gekennzeichnet ist, daß als perfluoriertes, verzweigtkettiges Olefin Perfluor-2-methyl-2-buten oder Perfluor-2-methyl-2-penten eingesetzt wird und die Umsetzung in Gegenwart von mindestens 1 mol je mol perfluoriertes Olefin eines löslichen Silbersalzes durchgeführt wird.

Es können verschiedene Molverhältnisse von lod und Alkalimetallfluorid angewendet werden, zweckmäßig benutzt man einen molaren Überschuß des Alkalimetallfluorids. Wegen des günstigen Preises werden bevorzugt Natriumfluorid oder Kaliumfluorid eingesetzt. Sofern das lösliche Silbersalz Silberfluorid ist, kann vorteilhaft auf die Verwendung des Alkalimetallfluorids verzichtet werden.

Als aprotisches Lösungsmittel sind beispielsweise geeignet Nitrile, zum Beispiel Acetonitril; Ether, z. B. Diethylenglykol-dimethylether oder Diethylenglykol-diethylether; Säureamide, z.B. Dimethylformamid oder N-methyl-acetamid sowie Nitroverbindungen, z. B. Nitrobenzol.

Das Mengenverhältnis des eingesetzten Perfluor-2-methyl-2-buten bzw. Perfluor-2-methyl-2-penten in bezug auf das verwendete Iod und Alkalimetallfluorid ist nicht kritisch. Da, wie weiter oben bereits erwähnt, die als Ausgangsprodukt verwendeten perfluorierten Olefine von den Verbindungen der Formel (I) durch Destillation leicht zu trennen sind, kann ein Überschuß der fluorierten Olefine angewendet werden, zweckmäßig werden jedoch äquimolare Mengen, bezogen auf Iod und Alkalimetallfluorid, eingesetzt. Das perfluorierte Olefin kann auch in weniger als der äquimolaren Menge angewendet werden.

Die Umsetzung findet in Gegenwart von mindestens 1 mol je mol perfluoriertes Olefin eines löslichen Silbersalzes statt. Wird weniger als 1 mol Silbersalz eingesetzt, so wird eine erhebliche Ausbeuteverminderung an Verbindungen der Formel (I) festgestellt. Die Menge des verwendeten Silbersalzes ist nach oben nur durch wirtschaftliche Erwägungen begrenzt, im allgemeinen sind 1 bis 2 mol Silbersalz je mol perfluoriertes Olefin völlig ausreichend.

Unter löslichen Silbersalzen sind solche zu verstehen, die zu mindestens 5 g pro $dm^3$ in Acetonitril bei 20 °C löslich sind. Geeignete Silbersalze sind beispielsweise die Carboxylate von Carbonsäuren, die keine reduzierende Wirkung haben und unter den Reaktionsbedingungen nicht mit Iod reagieren wie z. B. Essigsäure, ferner Silbertetrafluorborat oder -hexafluorphosphat, Silbersulfat oder Silbernitrit. Wegen ihrer besonders günstigen Wirkung sind Silbernitrat, Silbertrifluoracetat und Silberfluorid bevorzugt. Silberfluorid hat noch den weiteren Vorteil, daß die Anwendung von Alkalimetallfluorid entbehrlich wird.

Die in den unmittelbar vorangehenden Abschnitten beschriebene Umsetzung wird vorzugsweise bei 20 bis 100 °C durchgeführt. Unterhalb 20 °C wird im allgemeinen die Reaktionsdauer unnötig verlängert, oberhalb 100 °C ist wegen des mit steigender Temperatur zunehmenden autogenen Drucks der Reaktionsmischung eine unnötig aufwendige Apparatur erforderlich, auch nimmt die Bildung von unerwünschtem Hydroperfluoralkan zu. In der Regel ist es ausreichend, unter Atmosphärendruck zu arbeiten, wobei man zweckmäßig Rückflußbedingungen wählt.

Das vorstehend beschriebene Verfahren kann auch ohne aprotisches Lösungsmittel bei Temperaturen über 100 °C ausgeführt werden, allerdings werden hierbei deutlich schlechtere Ausbeuten festgestellt.

Die Reaktion muß mit weitgehend wasserfreien Ausgangsstoffen durchgeführt werden, während der Reaktion wird zweckmäßig in einer trockenen Schutzgasatmosphäre, beispielsweise Argon oder Stickstoff, gearbeitet.

Die Umsetzung ist in der Regel 0,5 bis 20 h nach Einstellung und Halten der Reaktionstemperatur abgeschlossen.

Vorzugsweise wird eine Reaktionsdauer von 1 bis 5 h gewählt.

Nach Beendigung der Reaktion werden die festen Anteile der Reaktionsmischung zweckmäßig unter Anwendung von leichtem Überdruck durch Filtration, beispielsweise auf einer Fritte, abgetrennt und mit einer kleinen Menge des verwendeten aprotischen Lösungsmittels gewaschen. Das Filtrat wird mit Wasser von 0 bis 10 °C versetzt, die gebildete organische Phase abgetrennt und erneut mit kaltem Wasser ausgeschüttelt. Schließlich wird die organische Phase mit einem geeigneten festen Trockenmittel, beispielsweise Magnesiumsulfat, möglichst wasserfrei gemacht und anschließend fraktioniert destilliert. Die Destillationstemperaturen der einzelnen Fraktionen sind weiter oben bereits beschrieben. Die Verbindungen der Formel (I) werden ebenfalls als violette Flüssigkeiten von hoher Reinheit erhalten.

Achtung! Wie bereits oben erwähnt, sind auch bei den zuletzt beschriebenen Verfahren wegen der Giftigkeit der eingesetzten Ausgangsstoffe und erhaltenen Endprodukte sorgfältige Schutzmaßnahmen erforderlich.

Aus dem Filterrückstand der zuletzt genannten Reaktion kann leicht durch Waschen mit Wasser das gebildete Silberiodid isoliert werden. Aus diesem wird zweckmäßig das Silber durch Reduktion, beispielsweise mit Hydrazin oder einem anderen geeigneten Reduktionsmittel, abgeschieden und aus dem verbleibenden Teil durch anschließende vorsichtige Oxidation, beispielsweise mit Wasserstoffperoxid, das Iod wiedergewonnen.

Das bei der Destillation als leichtsiedende Fraktion abgetrennte nicht-umgesetzte Perfluorolefin kann ohne weitere Reinigung wieder eingesetzt werden.

Die erfindungsgemäß hergestellten Verbindungen der Formel (I) sind sehr reaktiv und lassen sich z. B. bei 100 bis 150 °C ohne Katalysator an Olefine wie z.B. Ethen oder Tetrafluorethen, oder an Alkine wie z.B. Propin oder Phenylacetylen addieren. Aus den in hohen Ausbeuten zugänglichen Additionsverbindungen lassen sich nach üblichen Methoden eine Reihe von Produkten mit guter oberflächenaktiver Wirkung herstellen. Die Verbindungen der Formel (I) eignen sich außerdem besonders gut für die Verwendung in Iod-Photo-Dissoziationslasern, besonders bei Anregung durch Sonnenlicht (solargepumpte Laser). Für die

letztgenannte Anwendung sind Verbindungen erwünscht, deren Ultraviolett-Absorptionsmaximum bei 285 nm oder höher liegt und die möglichst geringe Neigung zur Bildung eines Dimeren nach Abdissoziation des Iods zeigen. Letztere Eigenschaft wird erreicht, wenn das Iod an einem tertiären Kohlenstoffatom eines perfluorierten Alkans gebunden ist. Dadurch erhöht sich allerdings die Giftigkeit der Produkte, so daß generell Verbindungen von Vorteil sind, die geringere Flüchtigkeit aufweisen. Dies ist bei den Produkten der Formel (I), verglichen mit dem bekannten 2-Iod-perfluormethyl-propan, der Fall. Außerdem zeigen die Verbindungen der Formel (I) besonders günstige Ultraviolett-Absorptionsmaxima, wie nachfolgende Tabelle I zeigt.

Tabelle I: UV-Daten von Iod-perfluoralkanen

| Verbindung | $\lambda_{max}$ (nm) | $\varepsilon$ |
|---|---|---|
| $CF_3-(CF_2)_5-I$ | 273,0 | 278 |
| 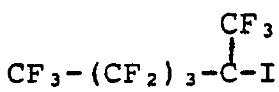 | 282,0 | 210 |
| 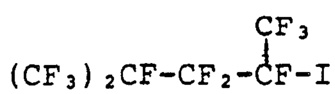 | 281,9 | 200 |
| 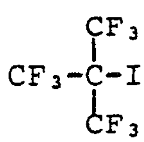 | 287,2 | 199 |
| 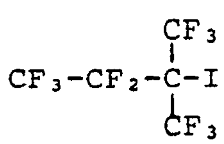 (IIa) | 292,6 | 190 |
| 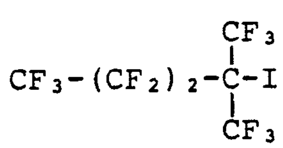 (IIb) | 294,6 | 196 |

Alle Messungen in n-Hexan.

$\lambda_{max}$ = Maximum der Absorptionsbande

$\varepsilon$ = molarer Extinktionskoeffizient.

Folgende Beispiele und Vergleichsversuche sollen die Erfindung näher erläutern:

Herstellung von 2-Iod-perfluor-2-methyl-butan

Beispiel 1

Ein Autoklav aus $V_2A$®-Stahl von 250 $cm^3$ Inhalt wird mit 5,8 g (0,1 mol) Kaliumfluorid-Pulver beschickt und zur vollständigen Trocknung im Vakuum 1 h auf 200 °C erhitzt. Nach Abkühlen auf Raumtemperatur werden unter trockener Argonatmosphäre 11,1 g (0,05 mol) Iodpentafluorid und 25,4 g (0,1 mol) Iod in den Autoklaven gegeben, auf -70 °C gekühlt, evakuiert und 62,5 g (0,25 mol) Perfluor-2-methyl-2-buten einkondensiert. Der Autoklav wird innerhalb von 2 h unter Schütteln auf 185 °C geheizt und 12 h bei dieser Temperatur geschüttelt. Nach Abkühlen auf Raumtemperatur wird in einem wirksamen Abzug langsam entspannt und die flüssigen Anteile im Autoklaven werden in 100 ml eiskalte 10 gew.-%ige wäßrige Natronlauge eingerührt. Die organische Phase wird mit eiskaltem Wasser gewaschen, mit wenig Magnesiumsulfat getrocknet und bei normalem Atmosphärendruck destilliert. Es werden als Hauptlauf 72,3 g einer violetten Flüssigkeit mit Kp 89 bis 90 °C (73 % Ausbeute, bezogen auf eingesetztes Perfluor-2-methyl-2-buten) erhalten. Nach $^{19}F$-Kernresonanzspektrum besteht das Produkt zu mindestens 99,5 % aus 2-Iod-perfluor-2-methyl-butan.

Beispiel 2

Ein mit 11,6 g (0,2 mol) Kaliumfluorid beschickter Vierhalskolben mit 250 $cm^3$ Inhalt wird im Vakuum auf 200 °C erhitzt, auf Raumtemperatur abgekühlt und mit Rührer, Thermometer und einem Trockeneiskühler ausgerüstet, an dessen oberem Ausgang ein mit Blaugel gefülltes Trockenrohr angebracht ist. Nun werden 100 $cm^3$ reines wasserfreies Acetonitril und 22,1 g (0,1 mol) Silber(I)-trifluoracetat zugegeben und der Kolben mit einer Septumkappe verschlossen. Nach Kühlung des Kolbeninhalts auf 10 °C werden mit Hilfe einer gekühlten Spritze 25,0 g (0,1 mol) Perfluor-2-methyl-2-buten durch das Septum in den Kolben injiziert. Die Mischung wird 1 h bei Raumtemperatur, dann 30 min bei 30 °C gerührt. Nach Abkühlen auf Raumtemperatur werden 25,4 g (0,1 mol) Iod zugegeben. Nun wird zunächst 1 h bei Raumtemperatur, dann 3 h unter Rückfluß gerührt und anschließend auf Raumtemperatur abgekühlt. Die festen Anteile (Kaliumfluorid, Kaliumtrifluoracetat und Silber(I)-iodid) werden durch Filtration unter leichtem Überdruck auf einer Fritte abgetrennt und mit etwas Acetonitril gewaschen. Das Filtrat wird mit Eiswasser verdünnt, die untere organische Phase abgetrennt, mit kaltem Wasser ausgeschüttelt, erneut abgetrennt und mit wenig Magnesiumsulfat getrocknet. Anschließend wird fraktioniert destilliert. Es werden als Hauptlauf 31,4 g Produkt erhalten (79,3 % Ausbeute, bezogen auf Perfluor-2-methyl-2-buten). Die Reinheit entspricht dem nach Beispiel 1 erzeugten Produkt.

Herstellung von 2-Iod-perfluor-2-methyl-pentan

Beispiel 3

120 g (0,4 mol) Perfluor-2-methyl-2-penten, 11,1 g (0,05 mol) Iodpentafluorid, 25,4 g (0,15 mol) Iod und 5,8 g (0,1 mol) Kaliumfluorid werden wie in Beispiel 1 beschrieben umgesetzt und aufgearbeitet, mit dem Unterschied, daß die Reaktion bei 190 °C während 15 h durchgeführt wird. Die fraktionierte Destillation bei Normaldruck ergibt 99,7 g einer violetten Flüssigkeit mit Kp 115 °C. Die Ausbeute beträgt 89,4 %, bezogen auf Iodpentafluorid, nach gaschromatographischer Untersuchung besteht das Produkt zu 99,5 % aus 2-Iod-perfluor-2-methyl-2-pentan. Bei 22 °C beträgt die Dichte des Produktes 2,14 g/$cm^3$, der Dampfdruck 2800 Pa.

Beispiel 4

0,3 mol Perfluor-2-methyl-2-penten, 0,05 mol Iodpentafluorid, 0,1 mol Iod und 0,1 mol Cäsiumfluorid werden, wie in Beispiel 1 beschrieben,umgesetzt und aufgearbeitet, jedoch mit dem Unterschied, daß die Reaktion bei 210 °C während 15 h durchgeführt wird. Es werden 38,1 g des in Beispiel 3 beschriebenen Produktes erhalten. Die Ausbeute beträgt 34,1 %, bezogen auf Iodpentafluorid.

Beispiel 5

30,0 g (0,1 mol) Perfluor-2-methyl-2-penten, 22,1 g (0,1 mol) Silber(I)-trifluoracetat und 11,6 g (0,2 mol) trockenes Kaliumfluorid werden in 100 $cm^3$ wasserfreiem reinem Acetonitril 1 h bei 30 °C gerührt. Nach Abkühlen auf Raumtemperatur und Zugabe von 25,4 g (0,1 mol) Iod wird die Mischung 1 h bei Raumtemperatur und anschließend 2,5 h unter Rückfluß gerührt. Die Aufarbeitung erfolgt wie in Beispiel 2 angegeben, jedoch wird auf die fraktionierte Destillation verzichtet, da bereits das gewaschene Rohprodukt nach dem $^{19}F$-Kernresonanzspektrum aus reinem 2-Iod-perfluor-2-methyl-2-pentan besteht. Die Ausbeute beträgt 42,4 g = 95 %, bezogen auf eingesetztes Perfluor-2-methyl-2-penten. Durch Waschen des Filterrückstandes mit Wasser und Trocknen werden 23,0 g Silber(I)-iodid erhalten (98 % Ausbeute, bezogen auf eingesetztes Silber(I)-trifluoracetat).

Beispiel 6

Es wird verfahren wie in Beispiel 5 beschrieben, jedoch werden anstelle von Silber(I)-trifluoracetat 17,0 g (0,1 mol) Silber(I)-nitrat eingesetzt. Die Ausbeute an 2-Iod-perfluor-2-methyl-2-pentan beträgt 75 %, bezogen auf eingesetztes Perfluorolefin, die Ausbeute an Silber(I)-iodid 94 %, bezogen auf eingesetztes Silber(I)-nitrat.

Beispiel 7

Es wird verfahren wie in Beispiel 5, jedoch werden anstelle von Silber(I)-trifluoracetat 12,7 g (0,1 mol) Silber(I)-fluorid eingesetzt, das Kaliumfluorid weggelassen und die Reaktion unter Ausschluß von Licht durchgeführt. Die Ausbeute an 2-Iod-perfluor-2-methyl-2-pentan beträgt 94 %, bezogen auf eingesetztes Perfluorolefin, die Ausbeute an Silber(I)-iodid beträgt 98,5 %, bezogen auf eingesetztes Silber(I)-fluorid.

In nachfolgender Tabelle II sind die Kernresonanzdaten des bekannten 2-Iod-perfluor-2-methyl-propan sowie der beiden erfindungsgemäß hergestellten 2-Iod-perfluor-alkane aufgeführt.

TABELLE II

| Verbindung | $^{13}C$: Chem. Verschiebung und ($^{13}C$, $^{19}F$)-Kopplungen | $^{19}F$: Chem. Verschiebung |
|---|---|---|
| 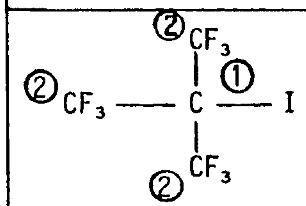 | ① 37,09 ppm, Dezett (30,5 Hz)<br>② 121,75 ppm, Quartett (290 Hz) | ② 14,45 ppm |
| 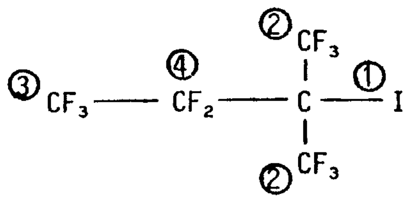 | ① 38,55 ppm, Septett (30,5 Hz) von Tripletts (25,2 Hz)<br>② 122,78 ppm, Quartett (286,8 Hz)<br>③ 118,34 ppm, Quartett (288,4 Hz) von Tripletts (35,3 Hz)<br>④ 113,02 ppm, Triplett (269 Hz) von Quartetts (40,4 Hz) | ② 16,16 ppm<br>③ 0,19 ppm<br>④ -24,84 ppm |
| 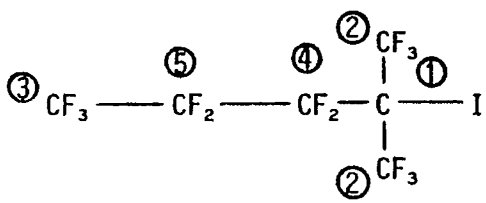 | ① 38,90 ppm, Septett (30,8 Hz) von Tripletts (24,6 Hz)<br>② 122,72 ppm, Quartett (286,5 Hz)<br>③ 118,61 ppm, Quartett (288,8 Hz) von Tripletts (34,1 Hz)<br>④ 114,68 ppm, Triplett (269,2 Hz) von Tripletts (34,0 Hz)<br>⑤ 109,78 ppm, Triplett (272,8 Hz) von Sextetts (37,7 Hz) | ② 16,36 ppm<br>③ - 3,06 ppm<br>④ -21,55 ppm<br>⑤ -43,30 ppm |

Alle Messungen in $CDCl_3$

$^{13}C$-NMR: 50,323 MHz, Tetramethylsilan als interner Standard

$^{19}F$-NMR: 75,393 MHz, Trifluoressigsäure als externer Standard

Vergleichsversuch A

In einen Autoklaven aus $V_2A$®-Stahl von 250 $cm^3$ Inhalt werden unter trockener Argon-Atmosphäre 0,05 mol Iodpentafluorid und 0,1 mol Iod gegeben, auf -70 °C gekühlt evakuiert und 0,3 mol Perfluor-2-methyl-2-buten einkondensiert. Der Autoklav wird innerhalb 2 h unter Schütteln auf 190 °C geheizt und 15 h bei dieser Temperatur geschüttelt. Nun wird weitergearbeitet, wie in Beispiel 1 beschrieben. Bei der fraktionierten Destillation wird im wesentlichen das Perfluorolefin zurückerhalten. Es hat sich offensichtlich kein 2-Iod-perfluor-2-methyl-butan gebildet.

Vergleichsversuch B

Es wird verfahren wie in Beispiel 1 beschrieben, jedoch werden anstelle von 0,1 mol nur 0,05 mol Kaliumfluorid und anstelle von 0,25 mol 0,3 mol Perfluor-2-methyl-2-buten eingesetzt. Die Reaktionstemperatur wird auf 200 °C eingestellt, die Reaktionsdauer beträgt 15 h. Bei der Aufarbeitung werden nur Spuren von 2-Iod-perfluor-2-methyl-butan erhalten.

Vergleichsversuch C

Es wird verfahren wie in Vergleichsversuch B, jedoch werden anstelle von 0,05 mol Kaliumfluorid 0,05 mol Antimon(V)-fluorid eingesetzt, die Reaktionstemperatur beträgt 210 °C. Es wird kein 2-Iod-perfluor-2-methyl-butan erhalten.

Vergleichsversuch D

Es wird verfahren wie in Vergleichsversuch B, jedoch werden anstelle von 0,05 mol Kaliumfluorid 0,1 mol Kobalt(III)-fluorid verwendet. Es wird kein 2-Iod-perfluor-2-methyl-butan erhalten.

Vergleichsversuch E

Analog E. P. Mochalina et al. Docl-Akad-Nauk SSSR 169 (6) Seite 1346 - 1349 (1966) werden 0,17 mol Perfluor-2-methyl-2-penten; 0,16 mol Iod; 0,34 mol Kaliumfluorid und 30 $cm^3$ Nitrobenzol während 10 h bei 175 °C umgesetzt. Bei der Aufarbeitung wird kein 2-Iod-perfluor-2-methyl-pentan erhalten.

Vergleichsversuch F

Es wird verfahren wie in Vergleichsversuch E, jedoch wird ohne Lösungsmittel (Nitrobenzol) gearbeitet und die Umsetzung während 15 h bei 200 °C durchgeführt. Auch hier wird kein 2-Iod-perfluor-2-methyl-2-pentan erhalten.

## Ansprüche

**1.** Verbindungen der Formel

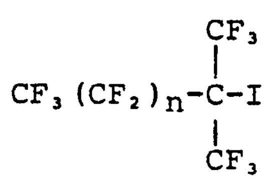

(I)

worin bedeutet n = 1 oder 2.

2. Verfahren zur Herstellung von Verbindungen der Formel (I) durch Umsetzung von perfluorierten, verzweigtkettigen Olefinen mit einer Mischung aus Iod und Iodpentafluorid im Molverhältnis von 2 : 1 bei erhöhter Temperatur unter dem autogenen Druck des Reaktionsgemisches oder einem darüberliegenden Druck, dadurch gekennzeichnet, daß als perfluoriertes, verzweigtkettiges Olefin Perfluor-2-methyl-2-buten oder Perfluor-2-methyl-2-penten in einer Menge von mindestens 5 mol je mol verwendetes Iodpentafluorid eingesetzt wird und die Umsetzung in Gegenwart von mehr als 1 mol je mol verwendetes Iodpentafluorid von mindestens einem Fluorid eines Metalls der I. Gruppe des Periodensystems der Elemente durchgeführt wird.

3. Verfahren nach Anspruch 2, dadurch gekennzeichnet, daß die Umsetzung bei 170 bis 220 °C durchgeführt wird.

4. Verfahren nach Anspruch 3, dadurch gekennzeichnet, daß die Umsetzung während 2 bis 30 h durchgeführt wird.

5. Verfahren nach einem oder mehreren der Ansprüche 2 bis 4, dadurch gekennzeichnet, daß die Umsetzung in Gegenwart von 1,1 bis 4,0 mol je mol verwendetes Iodpentafluorid von einem Fluorid eines Metalls der Gruppe I durchgeführt wird.

6. Verfahren zur Herstellung von Verbindungen der Formel (I) durch Umsetzung von perfluorierten, verzweigtkettigen Olefinen mit Iod und einem Alkalimetallfluorid in Gegenwart eines aprotischen Lösungsmittels, gegebenenfalls bei erhöhter Temperatur, dadurch gekennzeichnet, daß als perfluoriertes verzweigtkettiges Olefin Perfluor-2-methyl-2-buten oder Perfluor-2-methyl-2-penten eingesetzt wird und die Umsetzung in Gegenwart von mindestens 1 mol je mol perfluoriertes Olefin eines löslichen Silbersalzes durchgeführt wird.

7. Verfahren nach Anspruch 6, dadurch gekennzeichnet, daß die Umsetzung bei 20 bis 100 °C durchgeführt wird.

8. Verfahren nach Anspruch 7, dadurch gekennzeichnet, daß die Umsetzung während 0,5 bis 20 h durchgeführt wird.

9. Verfahren nach einem oder mehreren der Ansprüche 6 bis 8, dadurch gekennzeichnet, daß die Umsetzung ohne Alkalimetallfluorid und in Gegenwart von Silberfluorid durchgeführt wird.

10. Verwendung der Verbindungen der Formel (I) in Lasern.

## Claims

1. Compounds of the formula

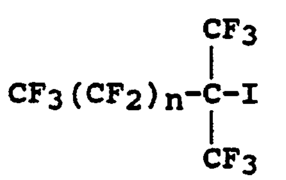

(I)

in which n denotes 1 or 2.

2. A process for the preparation of compounds of the formula (I) by reaction of perfluorinated branched-chain olefins with a mixture of iodine and iodine pentafluoride in a molar ratio of 2 : 1 at elevated temperature under the autogenous pressure of the reaction mixture, or a pressure above this pressure, characterised by using perfluoro-2-methyl-2-butene or perfluoro-2-methyl-2-pentene as the perfluorinated branched-chain olefin in an amount of at least 5 mol per mol of iodine pentafluoride used, and carrying out the reaction in the presence of more than 1 mol of at least one fluoride of a metal of group

I of the periodic table of the elements per mol of iodine pentafluoride used.

3. The process as claimed in claim 2, characterised in that the reaction is carried out at 170 to 220 °C.

4. The process as claimed in claim 3, characterised in that the reaction is carried out for 2 to 30 hours.

5. The process as claimed in any one of claims 2 to 4, characterised in that the reaction is carried out in the presence of 1.1 to 4.0 mol of a fluoride of a metal of group I per mol of iodine pentafluoride used.

6. A process for the preparation of compounds of the formula (I) by reaction of perfluorinated branched-chain olefins with iodine and an alkali metal fluoride in the presence of an aprotic solvent, if appropriate at elevated temperature, characterised by using perfluoro-2-methyl-2-butene or perfluoro-2-methyl-2-pentene as the perfluorinated branched-chain olefin and carrying out the reaction in the presence of at least 1 mol of a soluble silver salt per mol of perfluorinated olefin.

7. The process as claimed in claim 6, characterised in that the reaction is carried out at 20 to 100 °C.

8. The process as claimed in claim 7, characterised in that the reaction is carried out for 0.5 to 20 hours.

9. The process as claimed in any one of claims 6 to 8, characterised in that the reaction is carried out without an alkali metal fluoride and in the presence of silver fluoride.

10. The use of the compounds of the formula (I) in lasers.

**Revendications**

1. Composés répondant à la formule (I)

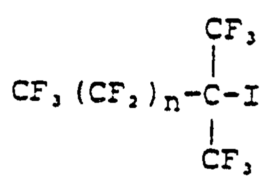

(I)

dans laquelle n est égal à 1 ou à 2.

2. Procédé pour préparer des composés de formule (I) par réaction d'oléfines ramifiées perfluorées avec un mélange d'iode et de pentafluorure d'iode dans un rapport molaire de 2:1, à température élevée et sous la pression autogène du mélange réactionnel ou sous une pression supérieure, procédé caractérisé en ce qu'on utilise, comme oléfine ramifiée perfluorée, le perfluoro méthyl-2 butène-2 ou le perfluoro méthyl-2 pentène-2 en une quantité d'au moins 5 mol par mole de pentafluorure d'iode utilisée, et on effectue la réaction en présence de plus d'l mol, par mole de pentafluorure d'iode utilisée, d'au moins un fluorure d'un métal du premier groupe de la classification périodique des éléments.

3. Procédé selon la revendication 2 caractérisé en ce qu'on effectue la réaction à une température de 170° à 220 °C.

4. Procédé selon la revendication 3 caractérisé en ce qu'on effectue la réaction pendant 2 à 30 h.

5. Procédé selon l'une quelconque des revendications 2 à 4, caractérisé en ce qu'on effectue la réaction en présence de 1,1 à 4,0 mol, par mole de pentafluorure d'iode utilisée, d'un fluorure d'un métal du groupe I.

6. Procédé pour préparer des composés de formule (I) par réaction d'oléfines ramifiées perfluorées avec l'iode et un fluorure de métal alcalin, en présence d'un solvant aprotique, éventuellement à température élevée, procédé caractérisé en ce qu'on utilise, comme oléfine ramifiée perfluorée, le perfluoro méthyl-2 butène-2 ou le perfluoro méthyl-2 pentène-2 et on effectue la réaction en présence d'au moins 1 mol

d'un sel d'argent soluble par mole d'oléfine perfluorée.

7. Procédé selon la revendication 6 caractérisé en ce qu'on effectue la réaction à une température comprise entre 20 et 100 °C.

8. Procédé selon la revendication 7 caractérisé en ce qu'on effectue la réaction pendant 0,5 à 20 h.

9. Procédé selon l'une quelconque des revendications 6 à 8, caractérisé en ce qu'on effectue la réaction sans fluorure de métal alcalin et en présence de fluorure d'argent.

10. Application des composés de formule (I) dans des lasers.